(19) Europäisches Patentamt
European Patent Office
Office européen des brevets

(11) Veröffentlichungsnummer : **0 262 126 B1**

(12) # EUROPÄISCHE PATENTSCHRIFT

(45) Veröffentlichungstag der Patentschrift : **03.07.91 Patentblatt 91/27**

(51) Int. Cl.$^5$ : **C12P 17/04**, C12P 31/00, C07D 307/93, C12R 1/72, C12R 1/84, C07C 49/242

(21) Anmeldenummer : **86903219.3**

(22) Anmeldetag : **16.05.86**

(86) Internationale Anmeldenummer : **PCT/DE86/00213**

(87) Internationale Veröffentlichungsnummer : **WO 86/07092 04.12.86 Gazette 86/26**

(54) **MIKROBIOLOGISCHE REDUKTION VON PROSTACYCLIN-ZWISCHENPRODUKTEN MIT EINER 15-KETOGRUPPE.**

(30) Priorität : **29.05.85 DE 3519548**

(43) Veröffentlichungstag der Anmeldung : **06.04.88 Patentblatt 88/14**

(45) Bekanntmachung des Hinweises auf die Patenterteilung : **03.07.91 Patentblatt 91/27**

(84) Benannte Vertragsstaaten : **AT BE CH DE FR GB IT LI LU NL SE**

(56) Entgegenhaltungen :
**EP-A- 0 012 710**
**Chemische Berichte, Band 103, 1970, Verlag Chemie GmbH, Weinheim (DE); J. Koch et al.:"Mikrobiologische Reduktion von 3-Keto-19-nor-5(10)-en-Steroiden zu entsprechenden 3-Hydroxy-Strukturen", S. 605, Tabelle 2**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **PETZOLDT, Karl**
**Flachsweg 10**
**W-1000 Berlin 38 (DE)**
Erfinder : **DAHL, Heinz**
**Gollanczstr. 102**
**W-1000 Berlin 28 (DE)**
Erfinder : **SKUBALLA, Werner**
**Olwenstr. 25**
**W-1000 Berlin 28 (DE)**

EP 0 262 126 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein mikrobiologisches Verfahren zur enantioselektiven Reduktion der 15-Ketofunktion in bicyclischen Prostacyclin-Zwischenprodukten zu den entsprechenden 15α-Hydroxy-Verbindugen.

Die Erfindung eignet sich besonders zur mikrobiologischen Reduktion der nachstehend aufgeführten Prostacyclin-Zwischenstufen 1 bis 6.

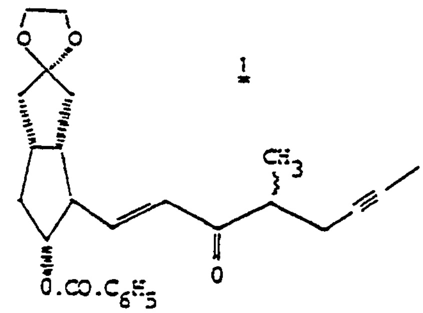

1

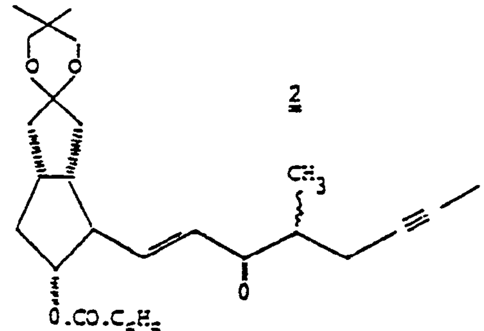

2

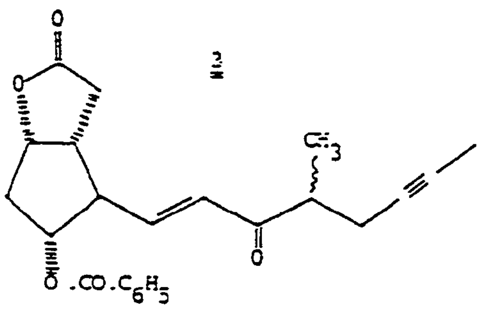

3

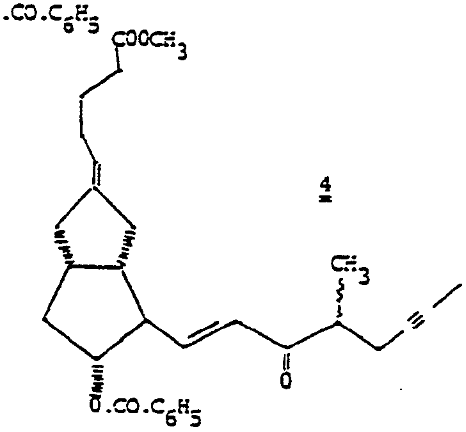

4

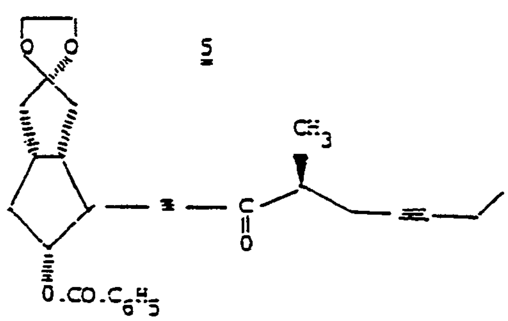

5

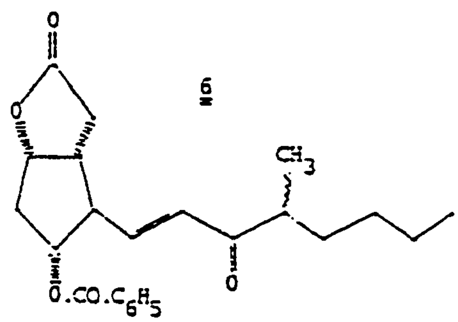

6

Die erfindungsgemäße mikrobiologische Reduktion an den vorstehend aufgeführten prostacyclin-Zwischenprodukten wird mit folgenden Mikroorganismen-Stämmen durchgeführt: Candida solani (NCYC 41), Pichia farinosa (NRRL-y-118) und Pichia farinosa (CBS 185). Besonders bewährt hat sich von diesen Stämmen der Stamm Candida solani.

Aus EP 12710 ist der nachfolgende Stand der Technik bekannt:

Während die chemische Reduktion der 15-Ketogruppen in Prostaglandinen und Prostaglandin-Zwischenprodukten zum Beispiel mit Natriumborhydrid nur über Gemische der entsprechenden 15α- und 15ß-Hydroxyverbindungen und wegen der sich anschließenden Trennung nur unter Ausbeuteverlusten zu den gewünschten 15α-Hydroxyprostaglandinen führt, sind durch die US-PS 3 687 811 eine Reihe von Mikroorganismen bekannt, die die 15-Ketogruppe in 11-Hydroxy-15-oxo-prostaglandinen je nach bereits vorhandener Konformation der 11-Hydroxygruppe in die entsprechende trans-15-Hydroxygruppe umwandeln. Mit dem Verfahren aus der DE-OS 23 57 815 verfügt der Stand der Technik über eine mikrobiologische Methode, die zum Beispiel ein 11-Hydroxy-15-oxo-prostaglandin in ein Gemisch aus 11α, 15α- und 11ß, 15ß-Dihydroxy-prostaglandin umwandelt. Die cis-Anordnung der 11α, 15α-Hydroxygruppen entspricht derjenigen in biologisch aktiven Prostaglandinen.

Die in der DE-OS 24 01 761 beschriebene Methode versagt ebenso wie alle genannten Verfahren.

Die in EP 12710 genannten Stämme Kloeckera, Saccharomyces und Hansenula reduzieren die 15-Ketogruppe in Prostacyclin-Zwischenprodukten nur teilweise und die Ausbeuten an Prostacyclin-Zwischenprodukten mit 15α-Hydroxygruppe sind sehr schlecht.

Es wurde nun gefunden, daß sich die oben genannten Prostacyclin-Zwischenstufen in sehr guten Ausbeuten enantioselektiv zu den entsprechenden 15α-Hydroxyverbindungen reduzieren lassen, wenn man dazu Candida- oder Pichia-Stämme verwendet.

Aus den nach dem erfindungsgemäßen Verfahren hergestellten 15α-Hydroxyverbindungen lassen sich unter Erhaltung des Asymmetriezentrums in 15-Position pharmakologisch wirksame Prostacycline herstellen. Beispielsweise gelangt gelangt man von (1S, 2S, 3R, 5R)-7,7-Ethylendioxy-3-benzoyloxy-2-[(1E), (4RS)-4-methyl-3-oxo-oct-1-en-6-in-yl]-bicyclio[3.3.0]octan (1) ausgehend in einer mehrstufigen Synthese zum Wirkstoff Iloprost (beschrieben in EP 11591)

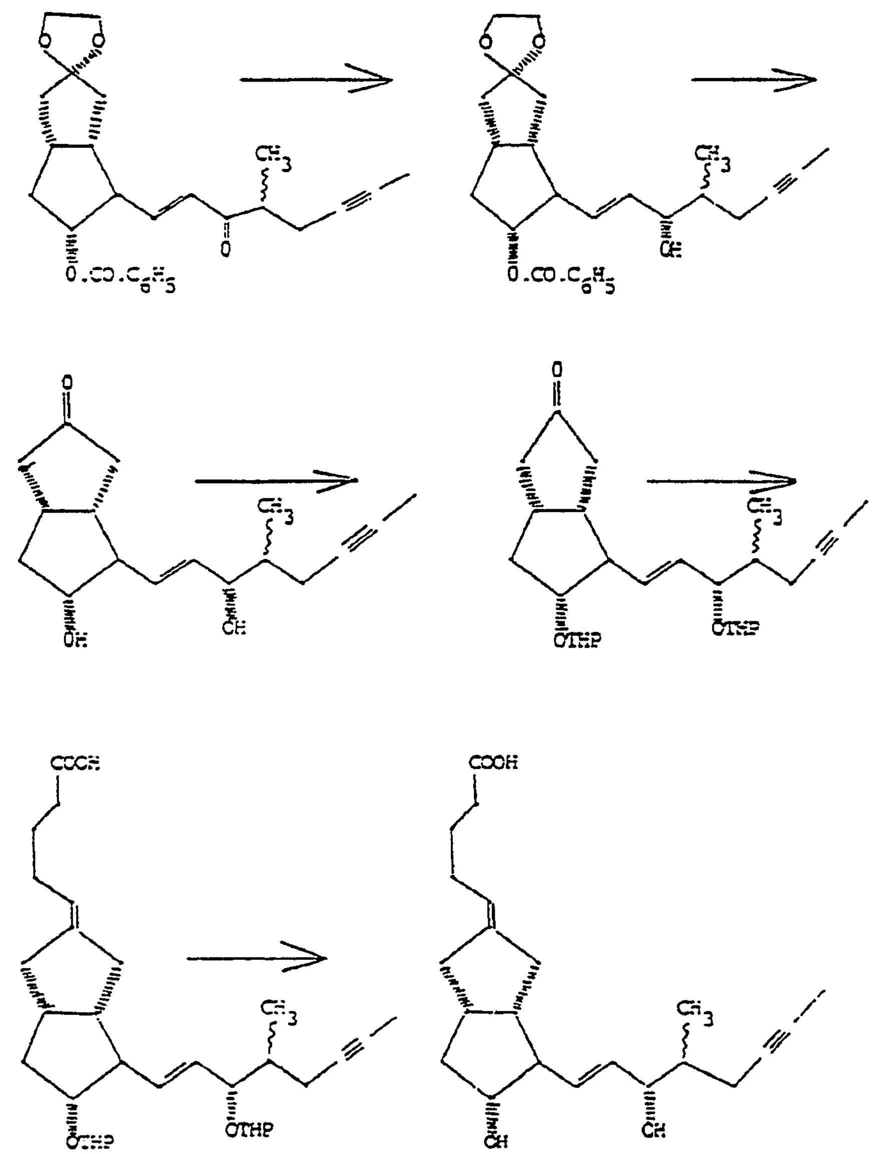

Unter den verschiedenen Arten der angegebenen Klassen von Mikroorganismen treten naturgemäß Unterschiede der Wirksamkeit in der erfindungsgemäßen Reduktion auf. Von Candida solani (NCYC 41), Pichia farinosa (NRRL-y-118) und Pichia farinosa (CBS 185), liegen gute Ergebnisse vor, wobei Reduktionen mit dem Stamm Candida solani (NCYC 41) besonders gute Ausbeuten ergeben.

Die Erfindung betrifft somit ein Verfahren zur enantioselektiven Reduktion der 15-Ketogruppe in bicyclischen Prostaglandin-Zwischenprodukten zu 15α-Hydroxyverbindungen der allgemeinen Formel

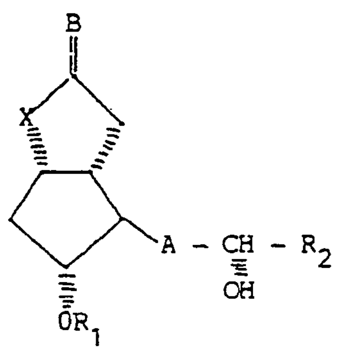

(I),

worin X Sauerstoff oder eine $CH_2$-Gruppe,
A eine trans –CH=CH– oder –C≡C-Gruppe,
B Sauerstoff, die Reste

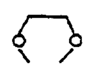

oder

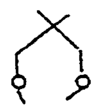

oder den

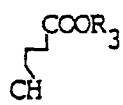

mit $R_3$ in der Bedeutung einer $C_1$-$C_4$-Alkylgruppe,
$R_1$ Wasserstoff, Tetrahydropyranyl, Benzoyl, tert.-Butyldimethylsilyl oder tert.-Butyldiphenylsilyl und
$R_2$ Reste

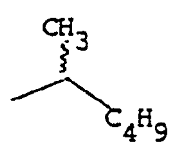

,

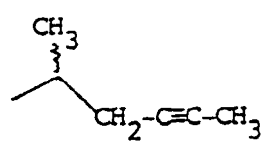

oder

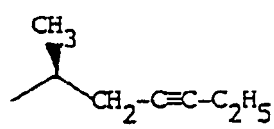

bedeuten, dadurch gekennzeichnet, daß man ein Keton der allgemeinen Formel II

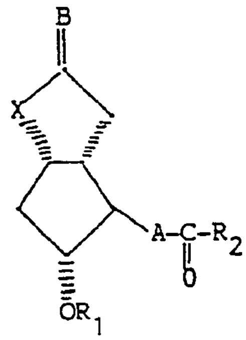

(II) ,

worin A, B, X, $R_1$ und $R_2$ die oben angegebene Beudeutung haben, mit Candida-solani- oder Pichia farinosa-Stämmen behandelt und das dabei entstehende 15-Hydroxy-Prostaglandin-Zwischenprodukt isoliert.

Unter $C_1$-$C_4$-Alkyl werden Methyl, Ethyl, Propyl, Isopropyl, Butyl, Isobutyl, sec.-Butyl und tert.-Butyl verstanden.

Besonders vorteilhaft arbeitet das Verfahren, wenn $R_1$ in Verbindungen der Formel II Benzoyl bedeutet.

Je nach der letzlich gewünschten Bedeutung von $R_1$ können die Schutzgruppen nach an sich bekannten Methoden abgespalten werden.

Zunächst werden unter den für die genannten Mikroorganismen üblicherweise verwendeten Kulturbedingungen in einem geeigneten Nährmedium und unter belüften Submerskulturen angezüchtet. Dann setzt man den Kulturen das Substrat (in einem geeigneten Lösungsmittel gelöst oder vorzugsweise in emulgierter Form) zu und fermentiert, bis eine maximale Substratumwandlung erreicht ist.

Geeignete Sübstratlösungsmittel sind beispielsweise Methanol, Äthanol, Glykolnmonomethyläther, Dimethylformamid oder Dimethylsülfoxyd. Die Emulgierung des Substrats kann beispielsweise bewirkt werden, in dem man dieses in mikronisierter Form oder in einem mit Wasser mischhabaren Lösungsmittel (wie Methanol, Äthanol, Aceton, Glykolmonomethyläther, Dimethylformamid, Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Äthylenoxydaddukte oder Fettsäureester von Polyglykolen. Als geeignete Emulgatoren seien die handelsüblichen Netzmittel Tegin(R), Tagat(R) und Span(R) beispielsweise genannt.

Oft ermöglicht die Emulgierung der Substrate einen erhöten Substratdurchsatz und somit eine Steigerung der Subtratskonzentration. Es is aber selbstverständlich auch möglich, bei dem erfindungsgemäßen Verfahren andere Methoden zur Steigerung des Substratdurchsatzes, wie sie dem Fermentationsfachmann wohl bekannt sind, anzuwenden.

Die optimale Substratkonzentration, Substratzugabezeit und Fermentationsdauer ist von der Struktur des verwendeten Substrates und der Art des verwendeten Mikroorganismus abhängig. Diese Größen müssen, wie dies bei mikrobiologischen Umwandlungen allgemein erforderlich ist, im Einzelfall durch Vorversuche, wie sie dem Fachmann geläufig sind, ermittelt werden.

Die Stämme Candida solani (NCYC 41) und

Pichia farinosa (CBS 185)

wurden bei der Deutschen Sammlung von Mikroorganismen unter den Nummern DSM 3315 und DSM 3316 am 21.05.1985 hinterlegt.

Die folgenden Ausführungsbeispiele dienen zur Erläuterung des erfindungsgemäßen Verfahrens.

## BEISPIEL 1

Ein 2 l-Erlenmeyerkoben, der 500 ml einer 30 Min. bei 120°C im Autoklaven sterilisierten Nährlösung, bestehend aus 1.5% Glucose-Monohydrat, 0,5% Yeast-Extract (Difco), 0,5% Corn steep liquor und 0,5% Ammoniumsulfat, pH auf 6,3 eingestellt, enthält, wird mit einer Schrägarkultur des Stammes Candida solani (NYCY 41) beimpft und 2 1/2 Tage auf einem Rotationsschüttler bei 30°C geschtüttelt.

Mit 250 ml dieser Anzuchtskultur wird eine 20 l-Vorfermenter beimpft, der mit 15 l eines 30 Min. bei 121°C und 1,1 bar Überdruck sterilisierten Nährmediums der gleichen Zusammensetzung wie die Anzuchtskultur beschickt ist. Unter Zugabe von Silicon SH als Antischaummittel wird bei 25°C und 0,7 bar Überdruck unter

Belüftung (15 l/Min.) und Rühren (220 U/Min.) 36 Stunden germiniert.

Danach werden 0,9 l dieser Vorfermenterkultur unter sterilen Bedingungen entnommen und damit ein 20 l-Hauptfermenter beimpft, der 10 l sterilisierte Nährlösung der gleichen Zusammensetzung wie die Vorfermenterkultur enthält. Nach einer Anwachsphase von 6 Stunden unter Vorfermenterbedingungen wird ein sterilisierte Lösung von 4 g (1S, 2S, 3R, 5R)-7,7-Ethylendioxy-3-benzoyloxy-2-[(1E), (4RS)-4-methyl-3-oxo-oct-1-en-6-in-yl]-bicyclo [3.3.0] octan gelöst in 100 ml Dimethylformamid, zugegeben und weiter gerührt und belüftet.

Nach 90 Stunden Kontaktzeit ist die Umsetzung beendet. Die Kulturbrüne wird 3 mal mit Methylisobutylketon extrahiert, die Extrakte vereinigt und i.V. zur Trockne eingeengt. Der ölige Rückstand wird in Methanol aufgenommen, vom Siliconöl abfiltriert und wieder zur Trockne eingedampft. Das hinterbliebene ölige Rohprodukt wird in Methylenchlorid gelöst und zur weiteren Reinigung über eine Kieselgelsaüle mittels eines Lösungsmittelgradienten 5 l Hexan/4 l Hexan + 1 l Aceton chromatographiert. Man erhält hierbei 2,75 g reines (1S, 2S, 3R, 5R)-7,7-Ethylendioxy-3-benzoyloxy-2-[(1E) ; (3S, 4RS)-3-hydroxy-4-methyl-oct-1-en-6-in-yl]-bicyclo[3.3.0]octan in Form eines farblosen Öls.

BEISPIEL 2

Unter den in Beispiel 1 angegebenen Bedingungen wird zur gewünschten Ketoreduktion der Stamm Pichia farinosa (NRRL-y-118) verwendet.

BEISPIEL 3

Unter den in Beispiel 1 angegebenen Bedingungen wird zur gewünschten Ketoreduktion der Stamm Pichia farinosa (CBS 185) verwendet.

BEISPIEL 4

Unter den in Beispiel 1 angegebenen Bedingungen wird die Verbindung 3 mit Hilfe des Stammes Candida solani (NCYC 41) zur entsprechenden 15α-Hydroxyverbindung reduziert.

BEISPIEL 5

Unter den in Beispiel 1 angegebenen Bedingungen wird die Verbindung 5 mit Hilfe des Stammes Candida solani (NCYC 41) zur entsprechenden 15α-Hydroxyverbindung reduziert.

## Ansprüche

1. Verfahren zur herstellung von 15α-Hydroxy-Prostaglandin-Zwischenprodukten der allgemeinen Formel

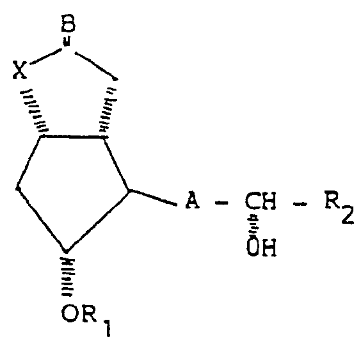

(I),

worin

X Sauerstoff oder eine $CH_2$-Gruppe,
A eine trans –CH=CH– oder –C≡C-Gruppe,
B Sauerstoff, die Reste

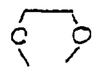 oder 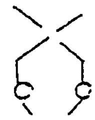

oder den

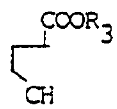

mit $R_3$ in der Bedeutung einer $C_1$-$C_4$-Alkylgruppe,

$R_1$ Wasserstoff, Tetrahydropyranyl, Benzoyl, tert.-Butyldimethylsilyl oder tert.-Butyl-diphenylsilyl und

$R_2$ die Reste

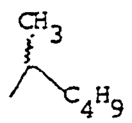 , 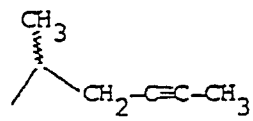 oder 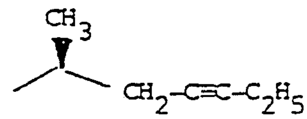

bedeuten, dadurch gekennzeichnet, daß man ein Keton der allgemeinen Formel II

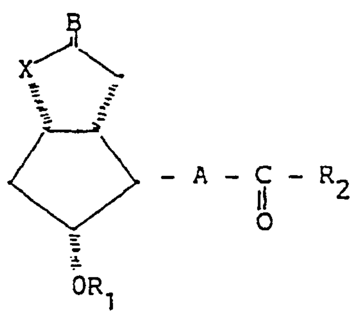 (II),

worin A, B, X, $R_1$ und $R_2$ die oben angegebene Bedeutung haben, mit Candida-solani- oder Pichia farinosa-Stämmen behandelt und das dabei entstehende 15-Hydroxy-Prostaglandin-Zwischenprodukt isoliert.

## Claims

1. Process for the preparation of 15α-hydroxyprostaglandin intermediates of the general formula

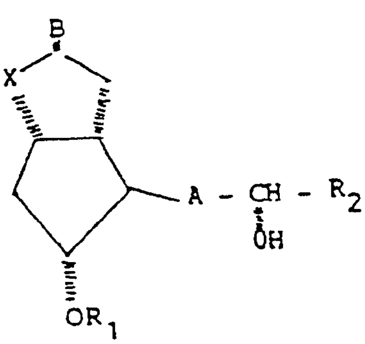 (I)

wherein

X represents oxygen or a $CH_2$ group,

A represents a trans –CH=CH– or –C≡C– group,
B represents oxygen, a radical

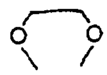 or 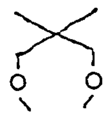

or the radical

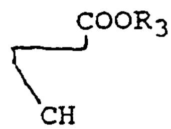

wherein $R_3$ represents a $C_1$-$C_4$-alkyl group,
$R_1$ represents hydrogen, tetrahydropyranyl, benzoyl, tert.-butyldimethylsilyl or tert.-butyldiphenylsilyl, and
$R_2$ represents a radical

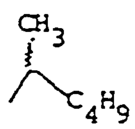 , 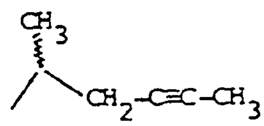 or 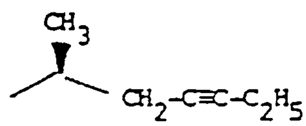 ,

characterised in that a ketone of the general formula II

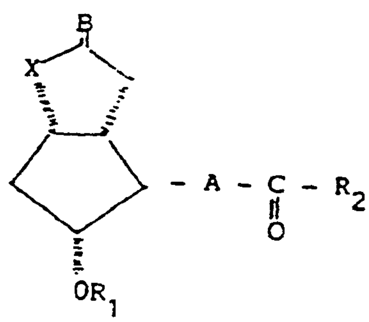 (II),

wherein A, B, X, $R_1$ and $R_2$ have the meaning given above, is treated with Candida solani or Pichia farinosa strains and the 15-hydroxyprostaglandin intermediate that forms is isolated.

## Revendications

1. Procédé pour préparer des corps intermédiaires pour prostaglandines à radical hydroxy en 15α qui répondent à la formule générale I :

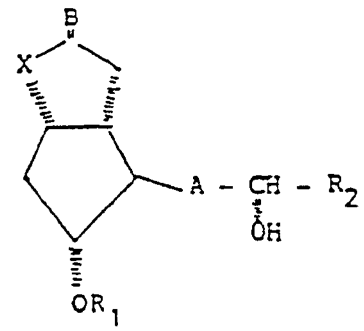

(I)

dans laquelle

X représente l'oxygène ou un radical $-CH_2-$,

A représente un radical $-CH=CH-$ trans ou un radical $-C\equiv C-$,

B représente l'oxygène, un radical

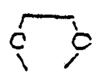 ou 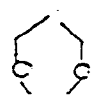 ,

ou un radical

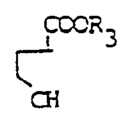

dont le symbole $R_3$ désigne un alkyle en $C_1$-$C_4$,

$R_1$ représente l'hydrogène ou un radical tétrahydropyrannyle, benzoyle, tert-butyldiméthyl-silyle ou tert-butyl-diphényl-silyle,

$R_2$ représente l'un des radicaux :

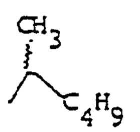 , 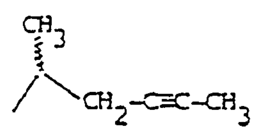 et 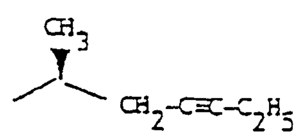 ,

procédé caractérisé en ce qu'on traite une cétone répondant à la formule générale II :

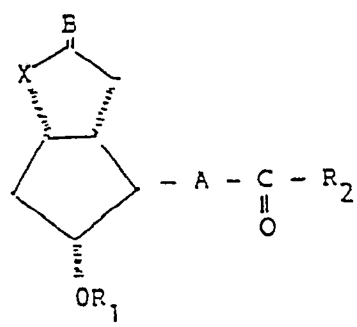

(II)

dans laquelle A, B, X, $R_1$ et $R_2$ ont les significations qui leur ont été données ci-dessus, par des souches Candida solani ou Pichia farinosa, et on isole le corps intermédiaire pour prostaglandines à hydroxy en 15 qui s'est ainsi formé.